# EUROPEAN PATENT SPECIFICATION

(11) **EP 1 768 728 B1**
(45) Date of publication and mention of the grant of the patent: **15.10.2008**
(21) Application number: 05754151.8
(22) Date of filing: 23.06.2005
(51) Int. Cl.: A61M 15/00, G06M 3/02

(54) **INHALER DEVICE COUNTER**
ZÄHLER FÜR EINE INHALATIONSVORRICHTUNG
COMPTEUR POUR INHALATEUR

(30) Priority: 05.07.2004 SE 0401787
(43) Date of publication of application: 04.04.2007
(73) Proprietor: AstraZeneca AB, 151 85 Södertälje (SE)
(72) Inventor: HODSON, Darren, AstraZeneca R & D Charnwood, Loughborough, Leicestershire LE11 5RH (GB); TRENEMAN, William, The Useful Product Company, Dry Drayton, Cambridgeshire CB3 8AT (GB); BUNCE, Martin, Marlborough, Wiltshire SN8 2AG (GB)
(74) Representative: Wahlström, Stig Christer Gunnar
(86) International application number: PCT/SE2005/000995
(87) International publication number: WO 2006/004497

(56) References cited:
- EP-A2- 0 381 494
- EP-A2- 0 684 047
- WO-A1-20/04089451
- US-A1- 2003 188 741

## Description

The present invention relates to the art of inhaler device counters, and in particular to a display arrangement for such and a method of providing such a display arrangement.

### Background of the Invention

Many types of medicines are provided in fluid form, such as a solution or suspension of particles in a propellant or emulsion, and are adapted for oral inhalation by a patient. As one example, a container might contain asthma medicine such as fluticasone propionate.

In order to deliver medicine to the patient, the can operates in conjunction with an actuator as a system commonly known as a metered dose inhaler (MDI) system. The actuator includes a housing having an open container-loading end and an open mouthpiece. A nozzle element is disposed within the housing and includes a valve stem-receiving bore communicating with a nozzle orifice. The orifice is aimed toward the mouthpiece. In order to receive a properly metered dosage of medicine from the container, the patient installs the container into the actuator through the container -loading end until the valve stem is fitted into the receiving bore of the nozzle element. With the container so installed, the opposite end of the container typically extends to some degree outside the actuator housing. The patient then places the mouthpiece into his or her mouth and pushes downwardly on the exposed container end. This action causes the container to displace downwardly with respect to the valve stem, which in turn unseats the valve. Owing to the design of the valve, the design of the nozzle element, and between the interior of the container and the ambient air, a short burst of precisely metered, atomized medicine is thereby delivered to the patient.

Such a container is filled with a predetermined volume of active substance, i.e. medicine. Hence, the container can nominally deliver a predetermined number of medicine doses before it has to be discarded. In order to visualize the number of remaining doses in such an inhaler device, it is preferably provided with a counter that displays the amount of medicine remaining in the container. Thus, the counter gives an indication of when to replace the inhaler device or container. The display of the "present state" can either be done in absolute terms, e.g. by showing in figures the actual number of doses that are still available, or in relative terms, e.g. by a color gradient from one color to another.

WO98/56444 to Glaxo Group Limited, shows a dose counter that is fixably secured on the outlet end of the aerosol canister and includes a display which denotes the number of metered doses of the medicament formulation left in the aerosol canister. The display of the dose counter is visible to the patient through a window provided in the actuator. The display is presented by a plurality of indicator wheels rotatably mounted on a common axle, each wheel having numerals from 0 to 9 displayed in series around the circumference. Before the dose counter is mounted on the aerosol canister, the display wheels are arranged so that the display shows the claimed total number of doses available in the aerosol canister.

US Patent No. 4817822 describes an aerosol dispenser of the type described above having a dose indicating device which, in a first embodiment is removably attached to the end of the protruding portion of the aerosol container. The operating mechanism of the dose counter is located within a housing which extends from the end of the aerosol container along the external surface of the tubular housing. The number of remaining doses in the container is indicated in relative terms by a colored strip that is incrementally withdrawn from an indicating window.

WO96/16686 describes an aerosol dispenser wherein the operating mechanism of the dose indicating device is electronic and wherein the actuating member comprises a microswitch set into the wall of the housing. The number of remaining doses is shown in absolute terms by an electronic display. However, electronic assemblies of this type are relatively expensive compared to equivalent mechanical mechanisms.

US5482030 describes an aerosol dispenser having a mechanical dose indicator device with angularly spaced number indications on a disc, which disc is rotated a predetermined angle for each actuation of the inhaler device.

US 2003/0188741 discloses an indicating device for aerosol containers, comprising a display disc with indices on the upper surface thereof. The indicia representing the present count state being displayed through a window. The display disc is incrementally rotated for e.g. every tenth actuation.

WO 2004/089451 which was filed before, but published after the filing date for the present application, discloses a counter for counting the number of doses delivered by a container.

The counter comprises a pointer unit of gear type that is in meshing relationship with two concentric gear wheels of different diameter, and which is brought to move along a display path by relative rotational movement of the two concentric gear wheels. The pointer wheel is viewable through a display window in the top surface of the counter and the number of doses available is indicated by indices provided adjacent to the window.

Exact indication of remaining number of doses in an inhaler device has an advantage of high accuracy, however the mere reading of a number, does not give a quick picture of the relative state with respect to the initial "full" state. Moreover it might be difficult to read, as the numbers have to be small sized in order to fit in the inhaler device.

Relative indication of remaining number of doses in an inhaler device, overcomes the problems with getting a quick picture of the relative state and the readability, as the appearance of the display can be made more intuitive and clear. However, it does not give an exact reading of the number of remaining doses, which is a major disadvantage especially when there is only a few number of doses left in the device.

In order to avoid particles (such as dust) or moisture to enter the counter, a transparent window is normally provided to cover the indicating section of the counter. Moreover, the window might prevent a user from tampering with the counter. In all prior art inhaler device counters, the transparent window is provided as an additional part, which has to be assembled with a counter housing.

### Summary of the Invention

The object of the invention is to provide a new inhaler device counter, which counter overcomes one or more drawbacks of the prior art. This is achieved by the inhaler device counter as defined in claim 1.

One advantage with such a counter is that it provides both a relative and exact indication of the remaining number of doses in an inhaler device.

Another advantage is that it is more rigid and wear resistant than prior art devices, as the counter window and the static display section are "integrated" in the molded part.

Embodiments of the invention are defined in the dependent claims.

### Brief Description of the Drawings

The invention will be described in detail below with reference to the drawings, in which
Fig. 1 is a schematic perspective view of an inhaler device with a counter according to the present invention.
Fig. 2 shows a schematic display arrangement for an inhaler device counter according to the present invention.
Fig. 3 is a schematic cross sectional view of the display arrangement of fig. 2
Fig. 4 is a schematic cross sectional view of a mould for moulding a top piece for an inhaler device counter according to the present invention.
Fig. 5 shows a label to be used in a "mould in label" process according to the present invention.
Fig. 6 is a schematic cross sectional view an alternative shape of a top piece for an inhaler device counter according to the present invention.

### Detailed Description of Preferred Embodiments

In order to combine the advantages of relative and exact indication of the remaining number of doses in an inhaler device counter, it has been found that indication using a display arrangement of pointer-gauge type gives an excellent combination of intuitive relative and exact reading of the number of remaining doses in an inhaler device. Moreover, it has been found that the design of the counter display arrangement can be enhanced by integrating the static part of the display arrangement in a transparent part of the counter housing. In one embodiment, the static part is integrated in the transparent part of the housing, utilizing an in mould label (IML) technique.

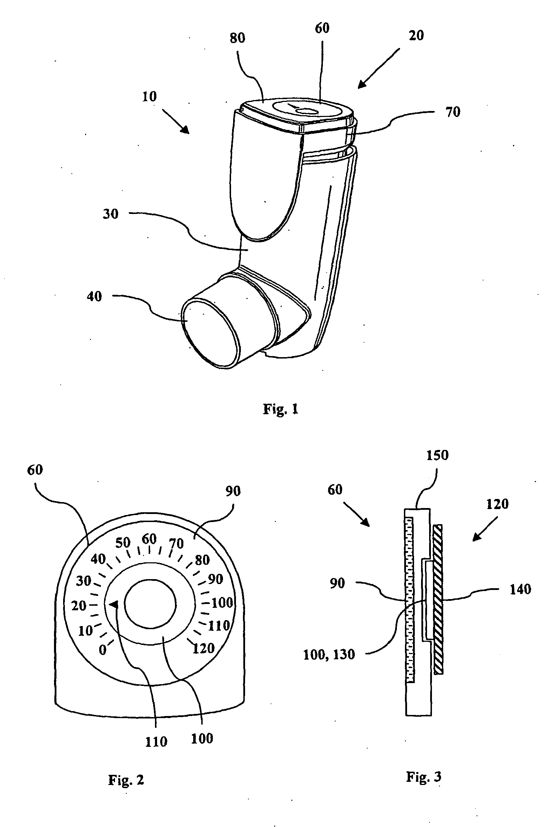

Fig. 1 shows a schematic example of an inhaler device 10 comprising a counter 20 according to the present invention. The inhaler device comprises an actuator body 30 with a mouth piece 40, through which medicine is delivered to the user, and a container-counter assembly. In this embodiment the counter 20 is attached to the end of an inhaler container (not shown) arranged in the actuator housing 30. The inhaler device 10 is actuated by depressing the container-counter assembly with respect to the actuator housing 30. The counter 20 is arranged to count each actuation of the inhaler device 10, and display the actual condition, via a display arrangement 60. The counter 20 could further be arranged as a part of, or being detachably attached to the actuator housing 30, e.g. on the front or back side thereof.

The counter 20 is basically comprised of a counter housing 70, a counter mechanism (not shown nor further described herein) and a display arrangement 60. In the embodiment shown in fig. 1 the display arrangement 60 is provided in a top surface 80 of the counter housing 70. In the disclosed embodiment, the top surface 80 of the housing 70 is provided as a transparent moulded part 150 (below referred to as display containing part 150) that closes the housing 70. In the disclosed embodiment, the counter top surface 80 further is utilized as an actuating surface for actuation of the inhaler device 10, i.e. for depressing the container-counter assembly. Because the counter top surface 80 is used as actuating surface, it has to be rigid and wear resistant, as it will be subjected to compressive force and wear during the actuation of the inhaler device 10.

Fig. 2 shows a schematic top view of the counter 20 with an example of the display arrangement 60 according to the present invention. The display arrangement 60 comprises a static display section 90 and a moveable display section 100. In the disclosed embodiment, the static display section 90 surrounds the moveable section 100, which is provided as a rotatable element with a pointer 110. The static display section 90 is an annular graduation area with indices for the number of doses remaining in the container, and the angular position of the pointer 110 thus gives the actual count. With this arrangement it is possible to achieve the advantages with both relative and exact value indication of the remaining number of doses.

Fig. 3 shows the essential parts of the display arrangement 60 of the counter 20 of fig. 2 in cross section. The moveable display section 100 is provided as a rotatable element 120, whose rotation is controlled by the counting mechanism (not shown). The rotatable element 120 comprises a display portion 130 with the pointer 110, and a counter mechanism engaging portion 140, such as a toothed wheel. In order to achieve a rigid and wear resistant outer surface of the display arrangement, the static display section 90 is provided as an integrated part of the transparent moulded part 150, hereafter referred to as the display containing part 150, of the counter housing 70, i.e. the display containing part 150 and the static display section 90 are provided as an unitary structure. The display containing part 150 of the counter housing 70 preferably is molded of transparent plastic material such as clear polypropylene.

In order to integrate the static display section 90 in the display containing part 150, in a reliable and efficient manner, an in-mould label technique (IML) is employed. The IML technique is widely used in the packaging industry, but it has not been used for labeling of inhaler devices, even though there are some major advantages of the technique. IML labels are extremely durable, as they basically are present as an integrated part of the molded piece. Moreover, the printing is protected by the transparent plastic layer, onto which the label is printed, whereby a user of an inhaler device 10 will not get in direct contact with the ink.

Further, the IML technique is well suited for mass production of molded articles, at low cost. The IML technique further allows use of complex multicolor labels, as the labels are printed on a large plastic sheet using a suitable high capacity printing technique before they are cut out from the sheet and integrated into the molded piece.

From surveys it has been found that the provision of a colored warning zone, to indicate that there are just a few numbers of doses left, in addition to the numbered scale is desirable. Due to the IML benefits technique, such colored and or complex images are relatively simple to provide.

The present invention also provides a method of providing an inhaler device counter display arrangement 60 for displaying the number of remaining doses in an inhaler device 10, comprising the steps:
- providing the transparent display containing part 150 of the counter housing 70, in which a static display section 90 is provided as an integrated part, the static display section 90 being a section of a display arrangement 60 of pointer-gauge type and
- providing the moveable display section 100 arranged to be incrementally moved by the counter mechanism.

In one embodiment the static display section 90 is provided as an integrated part of the display containing part 150 by use of an IML technique.

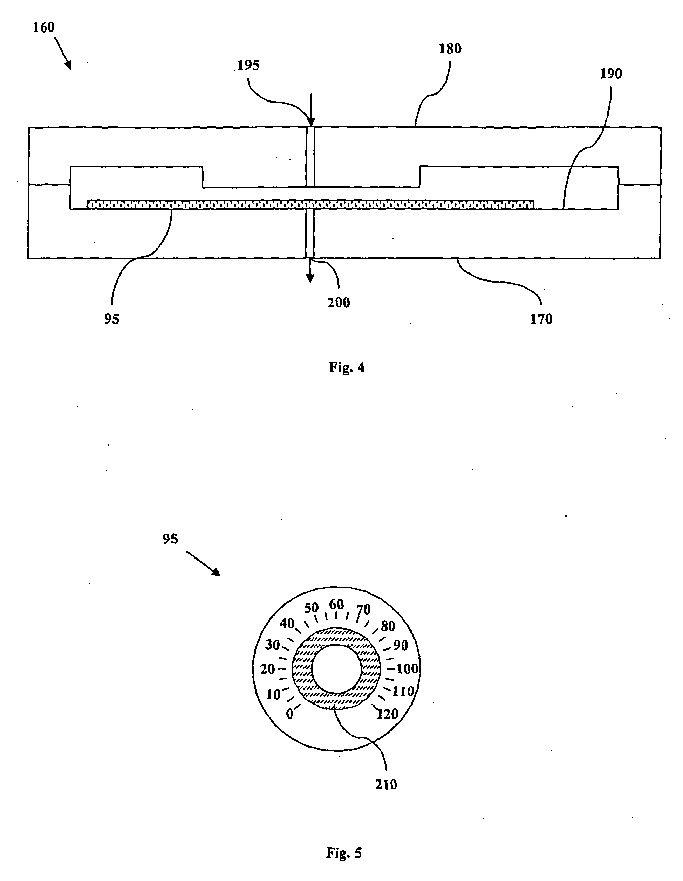

The IML process used to produce the display containing part 150 of the counter housing 70 according to the present invention will now be described with reference to fig. 4. Fig. 4 shows a schematic cross sectional view of a mold 160 for producing the display containing part 150 of the counter housing 70 with a static display section label 95 placed in the mold 160. The IML process comprises the following steps:
- providing a mold 160, defining a display containing part 150 of the counter housing 70, the mold 160 comprising a mold section 170 defining an outer surface of the display containing part 150 and at least one additional mold section 180,
- providing a static display section label 95 printed on a transparent plastic sheet,
- placing the static display section label 95 at a predetermined position on a surface 190 of the mold section 170 defining the outer surface of the display containing part 150 with the printed surface directed away from the mold surface 190,
- closing the mold 160,
- injecting a transparent plastic material through an injection port 195,
- hardening (or cooling) the plastic material,
- opening the mold 160 and removing the molded display containing part 150.

The static display section label 95 is so to say printed on the reverse side of thin sheet of plastic material. As the label is to be viewed through the thin sheet from the non reverse side, the printed label is a mirror image of the desired label. The plastic sheet is preferably of the same material as it will be integrated with.

In order to hold the label in position during the step of injecting plastic material, the mold can be provided with vacuum suction means as is indicated by 200 in fig. 4.

Fig. 5 shows the static display section label 95 for the display arrangement 60 of fig.2, wherein the dashed section 210 denotes a non-printed transparent section, intended to act as window for the pointer 110 shown in fig. 2.

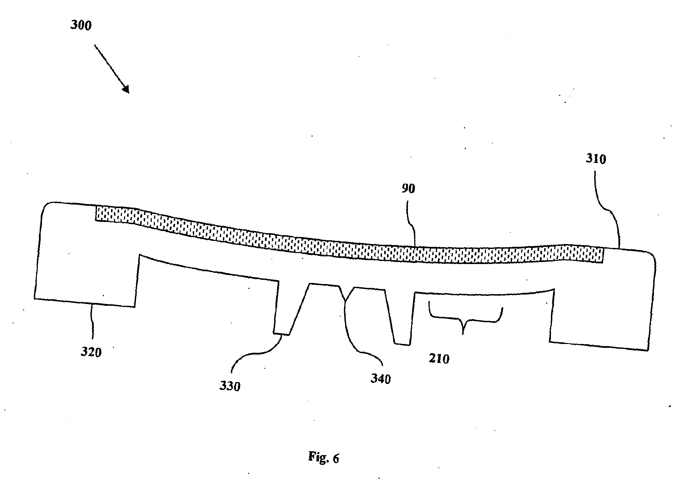

The above disclosed embodiment of the display containing part is in all aspects a schematic illustration. Fig. 6 shows a cross sectional view of a more detailed example of a display containing part 300. As is shown, the outer surface 310 of the display containing part 300 can be made curved while still using the IML technique, which might be advantageous when it is used as actuation surface as is the case in fig. 1. From fig. 6, it can be seen that the inner surface 320 of the display containing part 300 may have a complex shape, defining functional structures, e.g. of the counter mechanism. In fig. 6 the inside protrusions 330 are designed to function as a hub for the counter mechanism member carrying the rotatable display section. The tip 340 in the middle of the hub, represents the injection point for the plastic material. In order to achieve an appealing appearance of the display arrangement 60, the printed sections on the label 95 can be arranged to cover underlying structures that are not supposed to form a part of the display arrangement 60. In other words, only the section intended to act as window 210 for the pointer is left as a non printed transparent section.

From fig. 6 it is clear that it is not possible to print the static display section 90 onto the inside of the display containing part. Even if such a printing process was possible, it would be more expensive than the IML process, as it would require a single item printing technique, whereas the IML process preferably uses a cost effective continuous printing process.

## Claims

1. Inhaler device counter (20) for displaying the number of remaining doses in an inhaler device (10), the device counter comprising a counter housing (70) having a top surface (80), a counter mechanism and a display arrangement (60) of a pointer-gauge type with a static display section (90);
wherein
the static display section (90) comprises a static display section label (95) printed with ink on a transparent plastic sheet; and in that
the printing in the static display section (90) is protected by the transparent plastic sheet, onto which the label is printed, whereby a user of an inhaler device (10) will not get in direct contact with the ink.

2. Inhaler device counter (20) according to claim 1, **characterized in that** the static display section (90) is an annular graduation area with indices for the number of counts, and that the display arrangement (60) further comprises a moveable display section (100) in the form of a rotatable pointer (110).

3. Inhaler device counter (20) according to claim 1, **characterized in that** the static display section (90) surrounds a transparent section (210) that acts as a window for the moveable display section (100).

4. Inhaler device counter (20) according to claim 1, **characterized in that** the outer surface (310) of the display containing part (150, 300) is an actuating surface of the inhaler device (10).

5. Method of making an inhaler device counter for displaying the number of remaining doses in an inhaler device (10), **characterized by** the steps:
providing a transparent display containing part (150, 300) of a counter housing, in which a static display section (90) is provided as an integrated part, the static display section (90) being a section of a display arrangement (60) of pointer-gauge type, wherein the static display section (90) is provided as an integrated part of the display containing part (150, 300) by use of an in-mold label technique;
providing a moveable display section (100) arranged to be incrementally moved by a counter mechanism; and further comprising the steps of
providing a mould (160), defining a display containing part (150, 300) of the counter housing (70), the mould (160) comprising a mould section (170) defining an outer surface of the display containing part (150, 300) and at least one additional mould section (180),
providing a static display section label (95), printed on a transparent plastic sheet, placing the static display section label (95) at a predetermined position on the surface (190) of the mold section defining the outer surface of the display containing part (150, 300) with the printed surface directed away from the mould surface (190),
closing the mould (160),
injecting a transparent plastic material,
hardening (or cooling) the plastic material,
opening the mould (160) and removing the moulded display containing part (150, 300).

## Patentansprüche

1. Zähler (20) für eine Inhalationsvorrichtung zur Anzeige der Anzahl von verbleibenden Dosen in einer Inhalationsvorrichtung (10), wobei der Vorrichtungszähler ein Zählergehäuse (70) mit einer Oberseite (80), einen Zählermechanismus und eine Anzeigeanordnung (60) der Skalenzeigerart mit einem statischen Anzeigeabschnitt (90) aufweist;
wobei
der statische Anzeigeabschnitt (90) ein mit Tinte auf einer transparenten Kunststofffolie gedrucktes Etikett (95) des statischen Anzeigeabschnitts umfasst und
der Druck im statischen Anzeigeabschnitt (90) durch die transparente Kunststofffolie geschützt wird, auf die das Etikett gedruckt ist, wobei ein Benutzer der Inhalationsvorrichtung (10) mit der Tinte nicht in direkten Kontakt kommt.

2. Zähler (20) für eine Inhalationsvorrichtung nach Anspruch 1, **dadurch gekennzeichnet, dass** der statische Anzeigeabschnitt (90) ein ringförmiger Skaleneinteilungsbereich mit Indizes für die Anzahl von Zählungen ist und die Anzeigeanordnung (60) weiterhin einen beweglichen Anzeigeabschnitt (100) in Form eines drehbaren Zeigers (110) umfasst.

3. Zähler (20) für eine Inhalationsvorrichtung nach Anspruch 1, **dadurch gekennzeichnet, dass** der statische Anzeigeabschnitt (90) einen transparenten Abschnitt (210) umgibt, der als ein Fenster für den beweglichen Anzeigeabschnitt (100) dient.

4. Zähler (20) für eine Inhalationsvorrichtung nach Anspruch 1, **dadurch gekennzeichnet, dass** die Außenfläche (310) des die Anzeige enthaltenden Teils (150, 300) eine Betätigungsfläche der Inhalationsvorrichtung (10) ist.

5. Verfahren zur Herstellung eines Zählers für eine Inhalationsvorrichtung zur Anzeige der Anzahl verbleibender Dosen in einer Inhalationsvorrichtung (10), **gekennzeichnet durch** die folgenden Schritte:
Bereitstellen eines die Anzeige enthaltenden durchsichtigen Teils (150, 300) eines Zählergehäuses, in dem ein statischer Anzeigeabschnitt (90) als ein integrierter Teil vorgesehen ist, wobei der statische Anzeigeabschnitt (90) ein Abschnitt einer Anzeigeanordnung (60) der Skalenzeigerart ist, wobei der statische Anzeigeabschnitt (90) als ein integrierter Teil des die Anzeige enthaltenden Teils (150, 300) **durch** Verwendung einer In-Mould-Label-Etikettentechnik vorgesehen ist;
Bereitstellen eines beweglichen Anzeigeabschnitts (100), der zur inkrementalen Bewegung **durch** einen Zählermechanismus angeordnet ist; und weiterhin mit den folgenden Schritten:
Bereitstellen einer Form (160), die einen die Anzeige enthaltenden Teil (150, 300) des Zählergehäuses (70) definiert, wobei die Form (160) einen Formabschnitt (170), der eine Außenfläche des die Anzeige enthaltenden Teils (150, 300) definiert, und mindestens einen zusätzlichen Formabschnitt (180) umfasst,
Bereitstellen eines Etiketts (95) des statischen Anzeigeabschnitts, das auf einer transparenten Kunststofffolie gedruckt ist, wobei das Etikett (95) des statischen Anzeigeabschnitts in einer vorbestimmten Position auf der Fläche (190) des die Außenfläche des die Anzeige enthaltenden Teils (150, 300) definierendenFormabschnitts so platziert wird, dass die bedruckte Fläche von der Formfläche (190) weg weist,
Schließen der Form (160),
Einspritzen eines transparenten Kunststoffmaterials, Härten (oder Kühlen) des Kunststoffmaterials, Öffnen der Form (160) und Entfernen des geformten die Anzeige enthaltenden Teils (150, 300).

## Revendications

1. Compteur (20) pour dispositif inhalateur, destiné à afficher le nombre de doses restantes dans un dispositif inhalateur (10), le compteur pour dispositif comportant un boîtier (70) de compteur présentant une surface supérieure (80), un mécanisme de compteur et un agencement (60) d'affichage de type jauge à aiguille doté d'une portion (90) d'affichage statique ;
la portion (90) d'affichage statique comportant une étiquette (95) de portion d'affichage statique imprimée à l'encre sur une feuille de plastique transparent ; et l'impression de la portion (90) d'affichage statique étant protégée par la feuille de plastique transparent sur laquelle est imprimée l'étiquette, un utilisateur d'un dispositif inhalateur (10) n'entrant donc pas en contact direct avec l'encre.

2. Compteur (20) pour dispositif inhalateur selon la revendication 1, **caractérisé en ce que** la portion (90) d'affichage statique est une zone à graduation annulaire dotée d'indicateurs de nombre d'unités et **en ce que** l'agencement (60) d'affichage comporte en outre une portion (100) d'affichage mobile sous la forme d'une aiguille tournante (110).

3. Compteur (20) pour dispositif inhalateur selon la revendication 1, **caractérisé en ce que** la portion (90) d'affichage statique entoure une portion transparente (210) qui fait office de fenêtre pour la portion (100) d'affichage mobile.

4. Compteur (20) pour dispositif inhalateur selon la revendication 1, **caractérisé en ce que** la surface extérieure (310) de la partie (150, 300) contenant l'afficheur est une surface d'actionnement du dispositif inhalateur (10).

5. Procédé de fabrication d'un compteur pour dispositif inhalateur destiné à afficher le nombre de doses restantes dans un dispositif inhalateur (10), **caractérisé par** les étapes consistant à :
mettre en place une partie transparente (150, 300) contenant l'afficheur d'un boîtier de compteur, dans laquelle une portion (90) d'affichage statique est installée en tant que composant intégré, la portion (90) d'affichage statique constituant une portion d'un agencement (60) d'affichage de type jauge à aiguille, la portion (90) d'affichage statique étant installée en tant que composant intégré de la partie (150, 300) contenant l'afficheur en utilisant une technique d'inclusion d'étiquette dans le moule ;
mettre en place une portion (100) d'affichage mobile disposée de façon à être déplacée de façon incrémentale par un mécanisme de compteur ; et comportant en outre les étapes consistant à :
mettre en place un moule (160) définissant une partie (150, 300) contenant l'afficheur du boîtier (70) de compteur, le moule (160) comportant une portion (170) de moule définissant une surface extérieure de la partie (150, 300) contenant l'afficheur et au moins une portion (180) de moule supplémentaire,
mettre en place une étiquette (95) de portion d'affichage statique, imprimée sur une feuille de plastique transparent, placer l'étiquette (95) de portion d'affichage statique dans une position prédéterminée sur la surface (190) de la portion de moule définissant la surface extérieure de la partie (150, 300) contenant l'afficheur, la surface imprimée étant orientée à l'opposé de la surface (190) du moule, fermer le moule (160),
injecter une matière plastique transparente,
durcir (ou refroidir) la matière plastique,
ouvrir le moule (160) et extraire la partie moulée (150, 300) contenant l'afficheur.
